# EUROPEAN PATENT SPECIFICATION

(11) **EP 1 788 090 B1**
(45) Date of publication and mention of the grant of the patent: **25.04.2012**
(21) Application number: 06020842.8
(22) Date of filing: 04.10.2006
(51) Int. Cl.: C12N 15/70

(54) **Method for preparing artificial chromosomes which are free of host DNA**
Verfahren zur Herstellung künstlicher Chromosomen, die frei von DNA des Wirtes sind
Méthode pour la production de chromosomes artificiels sans l'ADN hôte génomique

(30) Priority: 04.10.2005 EP 05021667
(43) Date of publication of application: 23.05.2007
(73) Proprietor: Universität Regensburg, 93053 Regensburg (DE)
(72) Inventor: Klein, Christoph, 81373 München (DE); Schmidt-Kittler, Oleg, Baltimore MD 21214 (US); Stoecklein, Nick, 40489 Düsseldorf (DE); Fuhrmann, Christine, 81373 München (DE)
(74) Representative: Vossius & Partner

(56) References cited:
- FRENGEN E ET AL: "A Modular, Positive Selection Bacterial Artificial Chromosome Vector with Multiple Cloning Sites" GENOMICS, ACADEMIC PRESS, SAN DIEGO, US, vol. 58, no. 3, 15 June 1999 (1999-06-15), pages 250-253, XP004444891 ISSN: 0888-7543
- ZENG C ET AL: "Large-Insert BAC/YAC Libraries for Selective Re-isolation of Genomic Regions by Homologous Recombination in Yeast" GENOMICS, ACADEMIC PRESS, SAN DIEGO, US, vol. 77, no. 1-2, September 2001 (2001-09), pages 27-34, XP004432175 ISSN: 0888-7543
- PINKEL D ET AL: "HIGH RESOLUTION ANALYSIS OF DNA COPY NUMBER VARIATION USING COMPARATIVE GENOMIC HYBRIDIZATION TO MICROARRAYS" NATURE GENETICS, NEW YORK, NY, US, vol. 20, October 1998 (1998-10), pages 207-211, XP002925115 ISSN: 1061-4036
- ASHWORTH LINDA K ET AL: "Assembly of high-resolution bacterial artificial chromosome, P1-derived artificial chromosome, and cosmid contigs" ANALYTICAL BIOCHEMISTRY, vol. 224, no. 2, 1995, pages 564-571, XP002377945 ISSN: 0003-2697

## Description

The present invention relates to a novel method for isolating artificial chromosomes which are free of host DNA from a library, preferably BAC clones. The present invention further relates to various applications of said method in DNA analysis for medical, forensic, diagnostic or scientific purposes, e.g., ex vivo analyses of diseases like cancer being based on few or even single cells by, for example, microarray comparative genomic hybridization (CGH).

The enormous heterogeneity of malignant cells within a tumor facilitates tumor progression and often is the underlying cause of treatment failure. Unfortunately, our understanding of small cell populations or even single cells that promote tumor growth and are selected during the natural course of disease or by therapy is still limited. For example, cancer stem cells have been identified that display infinite self-renewal capacity while the majority of tumor cells is not clonogenic¹. Moreover, during metastatic progression significant differences of chromosomal aberrations between single disseminated tumor cells isolated from bone marrow of breast cancer patients and their matched primary tumors were uncovered². Because disseminated tumor cells are thought to comprise the precursor cells of later arising metastases³, they are particularly important for the understanding of systemic cancer progression. These two examples may stand for an increasing number of clinical and scientific questions that require techniques for genomic analyses of single cells.

Recently, microarrays have been developed, in which the metaphase chromosomes used for "Comparative Genomic Hybridization" (CGH) are replaced by clones of bacterial artificial chromosomes (BACs)^{4,5}, and array CGH chips covering the complete human genome at tiling resolution have become available⁶. It could be shown that the higher resolution of genomic analysis confers novel information about genes amplified or deleted in human cancer. However, while physical resolution could be increased 100 fold from metaphase CGH to array CGH, cellular resolution still is very poor, requiring 1 to 50 ng of high-quality DNA for the detection of single-copy number imbalances⁶, which limits the application of the technique to large tumors and cell lines. However, the analysis of a single cell would require a 200 to 1000 fold increase in cellular resolution - a goal that could so far not be attained.

Thus, the technical problem underlying the present invention is to provide methods for reliably analysing the genome of a small cell population or a single cell.

The solution to said technical problem is achieved by providing the embodiments characterized in the claims. During the experiments leading to the present invention it was investigated whether existing protocols of array CGH may be used to analyze DNA amplified from single cells or microdissected archival tumor samples. Previously, the present inventors reported a method for the amplification of DNA from a single cell or from microdissected, formalin-fixed and paraffin-embedded tissues, which retains the copy number ratios of chromosomes as determined by metaphase CGH^{7,8,24}. It was therefore tested how DNA amplified by this method can be analyzed on BAC arrays.

Here the first protocol for the preparation of BAC arrays that enables array CGH with DNA from single cells or from microdissected, formalin-fixed and paraffin-embedded tissues is described. This protocol allows detecting even copy number ratios of 3:2 in samples from single cells. For these applications previously published protocols resulted in enormous variations of hybridization signals due to non-human DNA contamination. The preparation of large arrays using the protocol described below enables a wide range of applications that require a detailed characterization of rare cells in cancer research or in medical genetics.

It was found that successful array CGH with DNA amplicons derived from single cells requires the DNA on the array to be essentially free of contaminating non-human DNA. Isolation of BAC DNA by pulsed field gel electrophoresis was the key to successful retrieval of array-CGH profiles from single cells. An additional effect of the subsequent amplification method was observed: adaptor-linker amplification was best suited for subsequent experiments. The protocol was extensively tested using single normal cells, single cells with known chromosomal aberrations (trisomy 21) and well-characterized single disseminated tumor cells isolated from the bone marrow of breast cancer patients. In addition, the purified BAC array was tested for the application of microdissected formalin-fixed paraffin-embedded tissues. All expected chromosomal aberrations could be detected by hybridization on PFGE-purified BACs while conventional arrays could only detect high copy number changes when single cells or minute amounts of DNA were analyzed.

In summary, the present invention, inter alia, allows to establish an array CGH protocol suited for numerous applications. Examples include the study of minute amounts of *ex vivo* DNA from premalignant lesions or very small tumors, from biopsies before and after systemic therapy, archived tumor samples of patients with known clinical outcome, analysis of single metastastic precursor cells or of circulating fetal cells in the maternal blood. Also, single cell analysis will be important in various fields studying the mechanisms of genetic instability of tumors or the genomic causes underlying human ageing^{17,18}, because most of these changes are stochastic in nature and require single cell resolution. Therefore, single cell array CGH using artificial chromosomes purified according to the method of the present invention will enable novel insights into important biological and medical questions.

Accordingly, the present invention relates to a method for isolating artificial chromosomes from a library of artificial chromosomes which are free of host DNA, said method comprising the following steps:
(a) preparing a library of artificial chromosomes with digested chromosomal DNA by use of a vector containing a recognition site for a restriction enzyme (x) which is not present within the genome of the host used for propagation of the artificial chromosomes;
(b) digesting the DNAs prepared from the library of artificial chromosomes of step (a) with restriction enzyme (x) after propagation of the library; and
(c) separating the digested linear artificial chromosomes of the library from the undigested circular host DNA by use of a separation method allowing to distinguish between linear and circular DNA.

Methods for preparing libraries of artificial chromosomes and suitable vectors which are useful in step (a) of the method of the present invention are known to the person skilled in the art and examples of such vectors are also provided in the appended examples. The term "preparing a library of artificial chromosomes" relates, accordingly, also to standard methods, as inter alia illustrated in Sambrook (2001) "Molecular Cloning: A Laboratory Manual", 3rd edition, Cold Spring Harbour Laboratory Press. Furthermore, and in accordance with this invention, already prepared libraries of artificial chromosomes may be employed. Such libraries comprise commercially available libraries care should be taken that the library used contains a recognition site for a restriction enzyme as defined in step (a) of the method of the invention.

Preferably, in the vectors useful in the method of the present invention, the recognition sequence for the restriction enzyme (x) is unique and digestion of the vector at this site does not interfere with important biological functions of the vector, e.g., as regards replication, selection markers etc..

General methods for isolating clones representing artificial chromosomes and for the preparation of libraries of artificial chromosomes e.g., YACs, BACs etc., from host cells and digesting the DNAs of the clones representing artificial chromosomes are well known to the person skilled in the art and, e.g., described in Sambrook and Russell (Molecular Cloning; A Laboratory Manual, 3rd Edition, Cold Spring Harbour Laboratory Press, Cold Spring Harbour, NY (2001)); see also Example 1(A) below. An example of a prepared library of artificial chromosomes is "FISH-mapped clones" V1.3; human 32 k BAC array from CHORI, USA.

Furthermore, the setting of conditions for the above described steps of the method of the present invention is well within the skill of the artisan and to be determined according to protocols described, for example in Sambrook et al, loc. cit., or in the appended examples.

As used herein, the term "free of host DNA" refers to isolated DNA, in particular artificial chromosomes that have been purified from host DNA contamination to such extent that it does not interfere with the methods of further analysis described in the sections below. Preferably, the content of contaminating host DNA is less than 3%, more preferably less than 1,5%, more preferably less than 1.0%, more preferably less than 0.6% and even more preferably less than 0.4% of the total DNA. The obtained "host cell-free" DNA is particularly useful in the preparation of microaarrays as documented in the appended examples and described herein below.

Preferred restriction enzymes (x) in the method of this invention are rare cutters like I-Ceul, PI-Scel, PI-Psal, I-Sal, Ascl and SgrAl with a sequence specificity of at least 9-10 or 11 bp, respectively. The restriction enzyme PI-Scel is particularly preferred. In a preferred embodiment of the method of the present invention, the library of artificial chromosomes is a library of BAC clones, e.g. the RPCI-11 library described in Osolgawa et al.²⁶. The generation of BAC libraries is, inter alia, described in reference ²⁷.

Any prokaryotic host cell can be used in the method of the invention, with bacterial host cells being preferred. Particularly preferred bacteria are *E*. *coli,* e.g., DH5a.

In a further preferred embodiment of the method of the present invention, the chromosomal DNA used for generating the library of artificial chromosomes is obtained from cells of a tissue sample, preferably from a human tissue sample.

The method of the present invention requires the efficient separation of linear DNA from circular nucleic (e.g., undigested host DNA). Such methods are known to the person skilled in the art with Pulsed-Field-Gel-Electrophoresis (PFGE) being preferred.

In a further preferred embodiment of the method of the present invention, in step (a) the chromsosomal DNA is isolated from cells which have no known numerical changes or imbalances in their DNA.

The present invention also relates to various uses of artificial chromosomes obtainable according to the herein provided method for isolating artificial chromosomes in a method for DNA analysis of a sample, comprising prenatal diagnosis, forensic medicine, pathogenic analysis or biological/biochemical research, wherein said method for DNA analysis comprises the following steps:
(a) preparing a microarray with the artificial chromosomes;
(b) hybridizing DNA obtained from (a) target cell(s) with the artificial chromosomes of said microarray; and
(c) evaluating the hybridization results,
wherein prior to step (b) the target DNA has been amplified, wherein said DNA has been obtained from a small cell population or a single cell and wherein steps (b) and (c) are performed as "Comparative Genomic Hybridization" (CGH).

Preferably, the isolated and linearized clones of the artificial chromosomes are presented as a microarray which can be prepared as, e.g., described in Example 1C.

Methods for carrying out steps (a), (b) and (c) are well known to the person skilled in the art. For example, the preparation of a microarray is described in Example 1 (C) or ref.^{22,23}. Prior to step (b) the target DNA has been amplified in order to enhance sensitivity of the assay, e.g., by PCR. Although PCR was initially applied to amplify a single locus in target DNA, it is increasingly being used to amplify multiple loci simultaneously. Frequently used primers for this general amplification of DNA are those based on repetitive sequences within the genome, which allow amplification of segments between suitable positioned repeats. Interspersed repetitive sequence PCR (IRS-PCR) has been used to create human chromosome- and region-specific libraries. A more general amplification than with ISR-PCR can be achieved with "degenerate oligonucleotide-primed PCR" (DOP-PCR), with the additional advantage of species independence. DOP-PCR is based on the principle of priming from short sequences specified by the 3'-end of partially degenerate oligonucleotides used, during initial low annealing temperature cycles of the PCR protocol. Since these short sequences occur frequently, amplification of target DNA proceeds at multiple loci simultaneously. DOP-PCR can be applied for generating libraries containing a high level of single-copy sequences, provided pure and a substantial amount of DNA of interest can be obtained, e. g. flow-sorted chromosomes, microdissected chromosome bands or isolated yeast artificial chromosomes (YACs). A method called "primer-extension preamplification" (PEP) is directed to circumvent this problem by making multiple copies of the DNA sequences present in a single cell. PEP uses a random mixture of 15-base fully degenerated oligonucleotides as primers, thereby leading to amplification of DNA sequences from randomly distributed sites. A method called representational difference analysis (RDA) is a subtractive DNA hybridization technique that discovers the differences between paired normal and tumor genomes. The minimal amount of DNA needed for RDA shown is 3 ng, corresponding to = 1 x 10³ cells; see for discussion of these amplification methods and corresponding references ref.²⁴. However, in the method of the present invention also single cells comprising about 6 pg DNA may be employed.

However, the most preferred amplification method for the assay of the present invention is the adapter-linker PCR which is capable of substantially uniform and preferably complete amplification of genomic DNA, particularly from a single cell, which is described in^{7,19,24}.

The target DNA to be analysed has been obtained from a small cell population or a single cell, most preferably a single cell. Thus, the DNA which is amplified is the genome of a small cell population or a single cell or chromosomes or (a) fragment(s) thereof. It has surprisingly been found that the method of the present invention is particularly useful for the analysis of single cells such as disseminating tumor cells, cells obtained from a lymph node, peripheral blood cells, cells from bone marrow aspirates, cells from tumor biopsis, cells obtained from microdissected tissue, or the like. The inventive method is particularly useful for the assessment of clonal evolution events of genetic variants in complex (cell) populations, like, inter alia, the clonal evolution of single micro-metastatic cells isolated from peripheral blood, bone marrow, or the like.

Accordingly, the methods provided herein or the uses of the artificial chromosomes (free of host cell DNA) are in particular useful for the study of minute amounts of *ex vivo* DNA from premalignant lesions or very small tumors, from biopsies before and after systemic therapy, archived tumor samples of patients with known clinical outcome, analysis of single metastastic precursor cells or of circulating fetal cells in the maternal blood. Accordingly, the methods/uses as provided herein also relate to the study of, e.g. biopsy samples, also samples obtained from liquor and tissues (like liver, brain, tissue of the gastro-intestinal tract, colon, rectum, tissue of the reproductive tract, uterus, bladder, ovary, prostate gland, testis, tissue from the alimentary canal, oesophagus, stomach, duodenum, ileum and jejunum, and the like). Most preferably, single cells derived from these tissues or samples are analyzed. Accordingly, the uses as described herein, e.g. the use of the obtained, host-cell DNA-free artificial chromosomes may be employed in a method for DNA analysis in a sample, for example ex vivo DNA from any cell in question or a cell expected to have chromosomal or subchromosomal inbalances/umbalances or structural genome aberrations.

In a particularly preferred embodiment of the use of the present invention, the methods for DNA analysis are comparative genomic hybridization (CGH)-, representational difference analysis (RDA)-, analytical PCR-, restriction enzyme length polymorphism analysis (RFLP)-, single strand conformation polymorphism analysis (SSCP)-, DNA sequence-, "loss of heterozygosity" (LOH)-, fingerprint-and/or FISH-analysis, i.e. the transition of a "normal" cell to a tumor cell may, in accordance with the method of this invention and the corresponding uses be monitored.

Analysis by CGH is the most preferred method. An important contribution of CGH to cancer research has been in pinpointing putative locations of cancer genes, especially at chromosomal sites undergoing DNA amplification. A large number of subregional chromosomal gains and DNA amplifications have been discovered by CGH in some cancers. Because oncogenes and drug-resistance genes are known to be upregulated by DNA amplifications, it has been speculated that DNA amplification sites in cancer could pinpoint locations of novel genes with important roles in cancer progression. Tumor progression implies the gradual transition of a localized, slow growing tumor to an invasive, metastatic and treatment refractory cancer. This progression is thought to be caused by a stepwise accumulation of genetic changes affecting critical genes. By providing genome-scale information of clonal genetic alternations, CGH is extremely useful in the analysis of the biological basis of the tumor progression process. The combination of the method/use of the present invention and CGH enables, e.g., the detection of DNA- copy number imbalances of single cells and thereby increases cellular resolution of array CGH at least 200 to 1000 fold.

Thus, in a further particularly preferred use the method for DNA analysis is a method for detecting numerical changes and/or imbalances in DNA of a sample, wherein said target cells are cells suspected to have numerical changes and/or imbalances in their DNA. Preferred examples of such target cells are tumor cells.

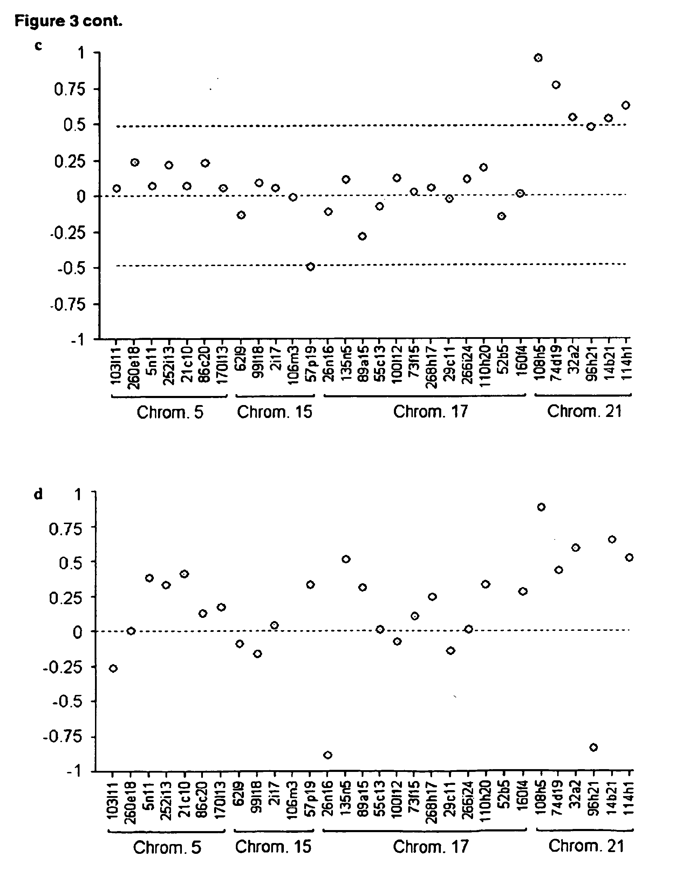

Finally, disclosed herein is a microarray with artificial chromosomes which are obtainable by the method of the present invention. This microarray is, in contrast to microarrays of the prior art free of host cell DNA. As shown in the appended examples, microarrays can be prepared with the herein described method which are microarrays for, inter alia, comparative genomic hybridization (CGH). It has to be pointed out that also Pinkel⁴ (1998) has prepared BAC chips. However, as documented in appended Figure 3d, this method of preparation (as described in Pinkel (1998) Nature Genetics 20, 207-211 is not suitable for the preparation of a single cell microarray/single cell array, i.e. single cell array CGH. In this invention it was successfully shown that this failure (based on the technique as described in Pinkel (1998)) was unexpectedly due to contamination of foreign host-DNA, i.e. bacterial DNA. In this invention it could be shown that said contaminating DNA precludes single cell array CGH. The method as provided herein for the isolation of artificial chromosomes and their corresponding use in e.g. microarrays overcomes these problems. Disclosed herein are microarrays which are free of host cell DNA. In this context "free of host cell DNA" means that less than 3% of the total DNA on said microarray is host DNA, e.g. bacterial DNA (like E. coli DNA). That means that the herein disclosed microarrays are, inter alia, useful in single cell CGH and comprise less than 3%, preferably less than 1.5% more preferably less than 1%, more preferably less than 0.6% even more preferably less than 0.4% contaminating host DNA (versus total DNA on said microarray).

### Brief description of the drawings

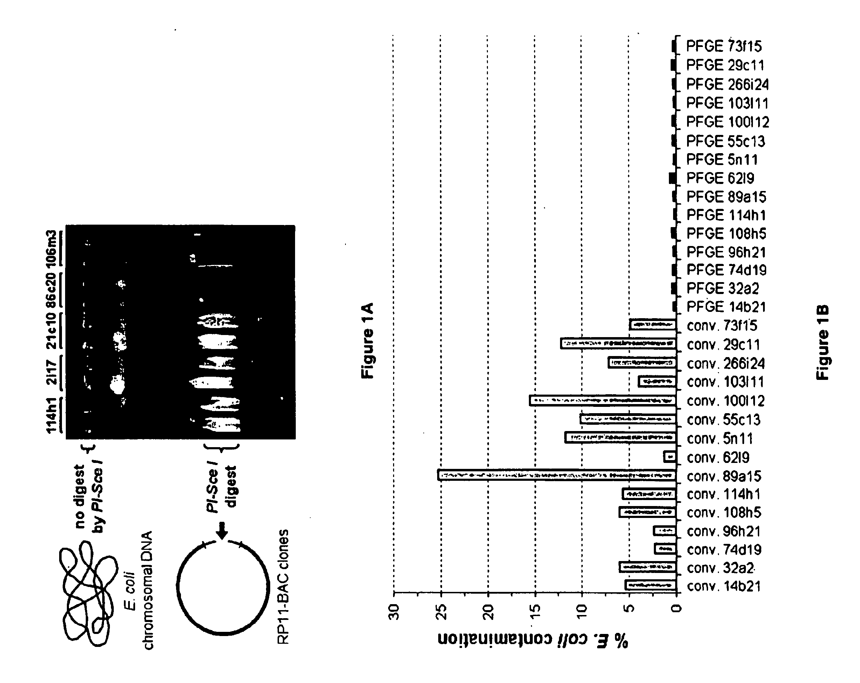

Figure 1: A Schematic illustration of the pulsed-field-ael-electrophoresis of *PI-Sce* / digested RP11-BAC clones
   **(A)** The *PI-SceI* site is located in the pBACe3.6 vector sequence, while no restriction site is present in the genomic DNA of *E. coli.* The linearized BAC-DNA is recovered after gel-electrophoresis by gel extraction (BAC clones 86c20, 106m3).
   **(B)** *E. coli* contamination of BAC clone preparations determined by qPCR. The amount of contamination is given as percentage of the total DNA (conv., conventional Qiagen preparation; PFGE, pulsed field gel electrophoresis purification of BAC clones).

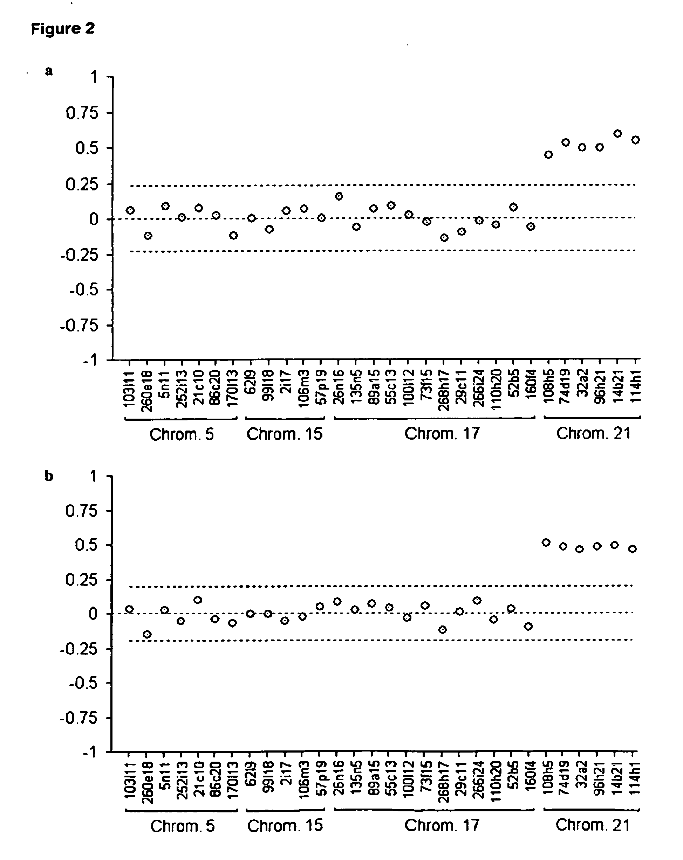

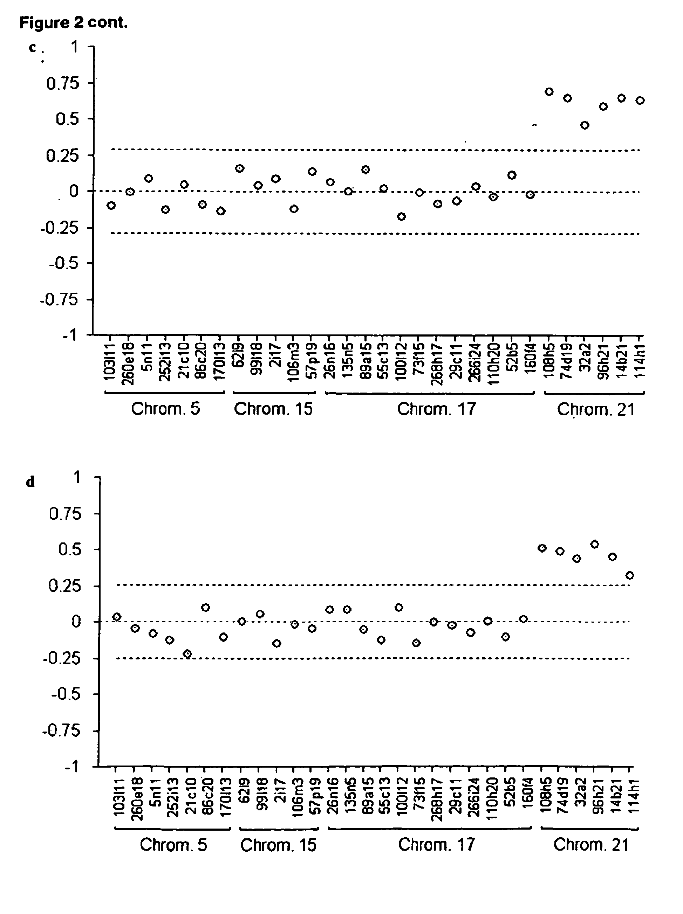

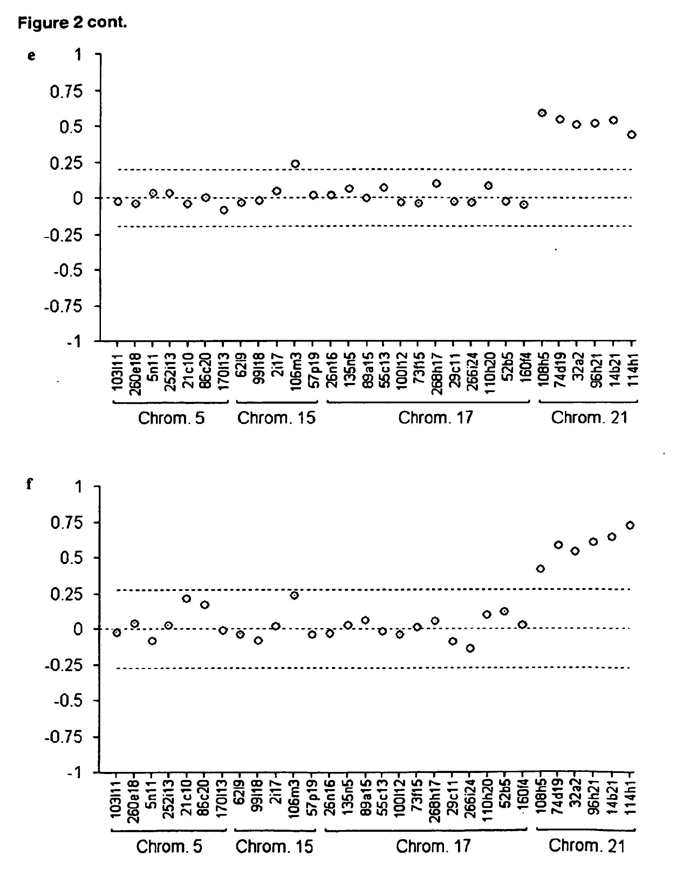

Figure 2: Hybridization of BAC microarrays using 0.5 µg DNA from a patient with trisomy 21
   The six different DNA preparations spotted onto one array were simultaneously hybridized with Cy3 and Cy5 labeled reference and test DNA probes. Trisomy 21 was detected by all protocols. The log₂ transformed ratios of test to reference DNA fluorescence intensities are plotted against the chromosomal locations of the BAC clones. Dashed gray and black lines represent the base line and the upper/lower significance thresholds defined by the three-times standard deviation.
   **a-c,** PFGE purification of BAC clones; **d-f,** Qiagen preparation of BAC clone;. **a** and **d,** Adapter-linker PCR; **b** and **e,** DOP-PCR; **c** and **f**, Phi29 rolling circle amplification.

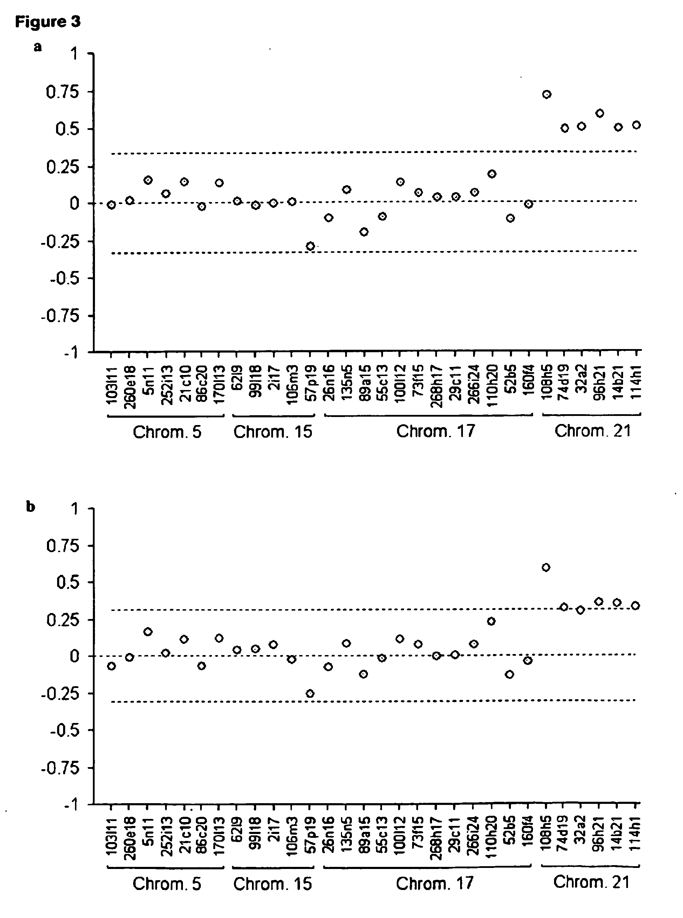

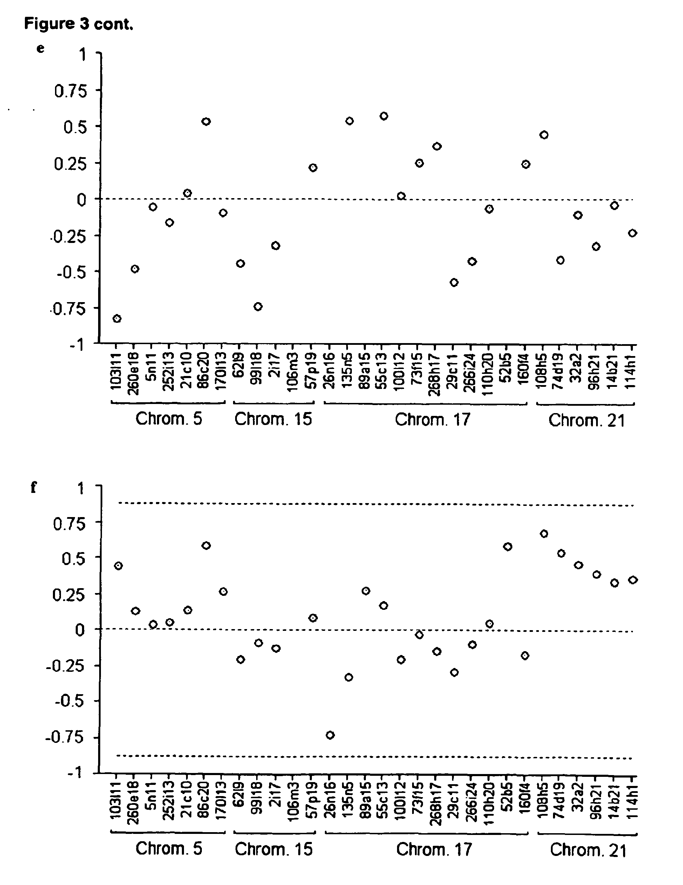

Figure 3: Hybridization of globally amplified DNA from a single PBL with trisomy 21
   The gain of chromosome 21 is only revealed by the hybridization on the PFGE purified BAC clone sub-arrays. Furthermore, the adapter-linker PCR amplified BAC clones show superior hybridization characteristics compared to the DOP or Phi29 amplified targets.
   **a-c,** PFGE purification of BAC clones; **d-f,** Qiagen preparation of BAC clones. **a** and **d**, Adapter-linker PCR; **b** and **e**, DOP-PCR; **c** and **f**, Phi29 rolling circle amplification.

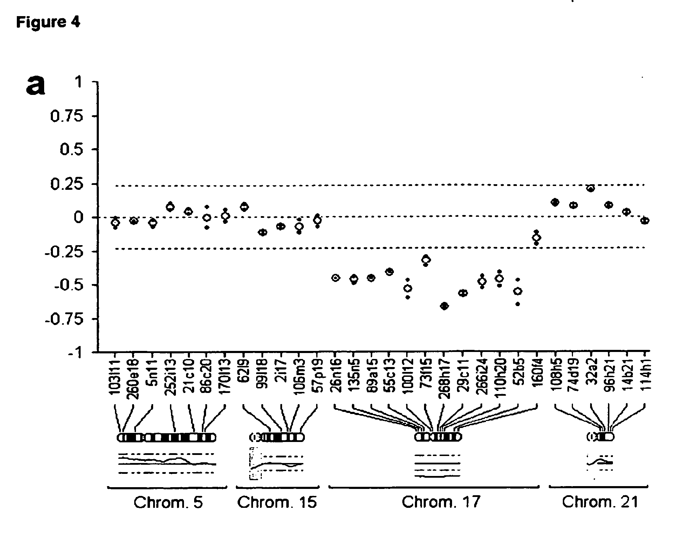

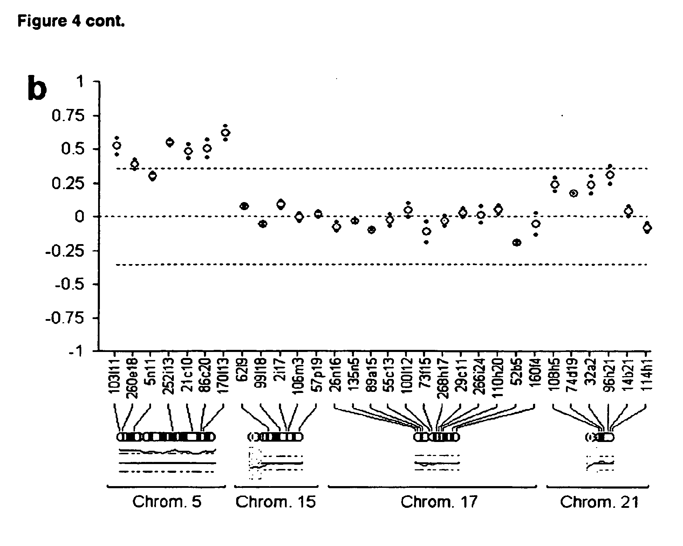

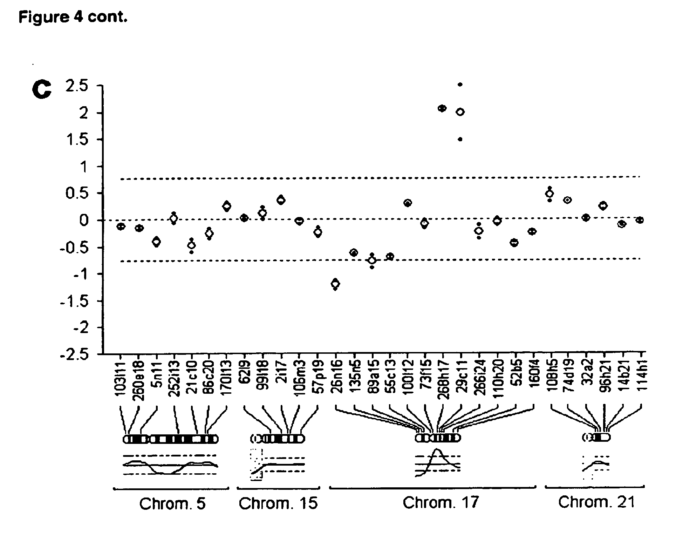

Figure 4: Hybridization of adapter-linker amplified DNA from single tumor cells and from microdissected paraffin embedded tumor tissue
   The hybridization profiles of the tumor samples on the PFGE purified, adapter-linker amplified BAC clone arrays is supplemented with the chromosomal locations of the probed BAC clones and the hybridization pattern of the same samples in metaphase CGH.
   **a** and **b**, Hybridization of globally amplified DNA from the single tumor cells TC-1 and TC-2 derived from bone marrow samples of two patients with mammary carcinoma. A copy number gain of chromosome 5 is detected in TC-2 and a deletion of chromosome 17 in TC-1.
   **c,** Hybridization of micro-dissected and globally amplified DNA from a formalin-fixed, paraffin-embedded pancreatic tumor sample. Note the Her2 amplification on chromosome 17q and the deletion of chromosome 17p. The log₂ transformed test to reference DNA fluorescence intensities rations are plotted against the chromosomal locations of the BAC clones. Dashed grey and black lines represent the base line and the upper/lower three times standard deviation significance thresholds, respectively. The green and red dots indicate the variance of the hybridization in color switch experiments.

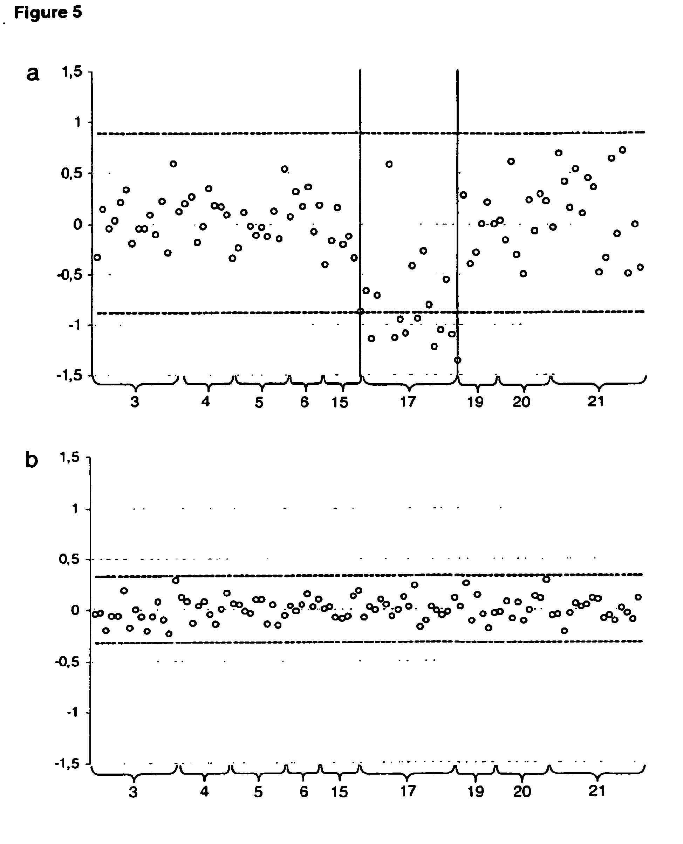

Figure 5: Hybridization of adapter-linker amplified DNA from normal single cells and single tumor cells
   Below the graph, chromosomal localization is indicated.
   **a,** Hybridization of globally amplified DNA from the single tumor cell TC-1 derived from bone marrow samples of one patient with mammary carcinoma. A deletion of chromosome 17 is detected in TC-1.
   **b,** Hybridization profile of two single cells derived from different normal donors. No deletions or amplifications can be detected. The log₂ transformed test to reference DNA fluorescence intensities rations are plotted against the chromosomal locations of the BAC clones. Dashed grey and blue lines represent the base line and the upper/lower three times standard deviation significance thresholds, respectively.

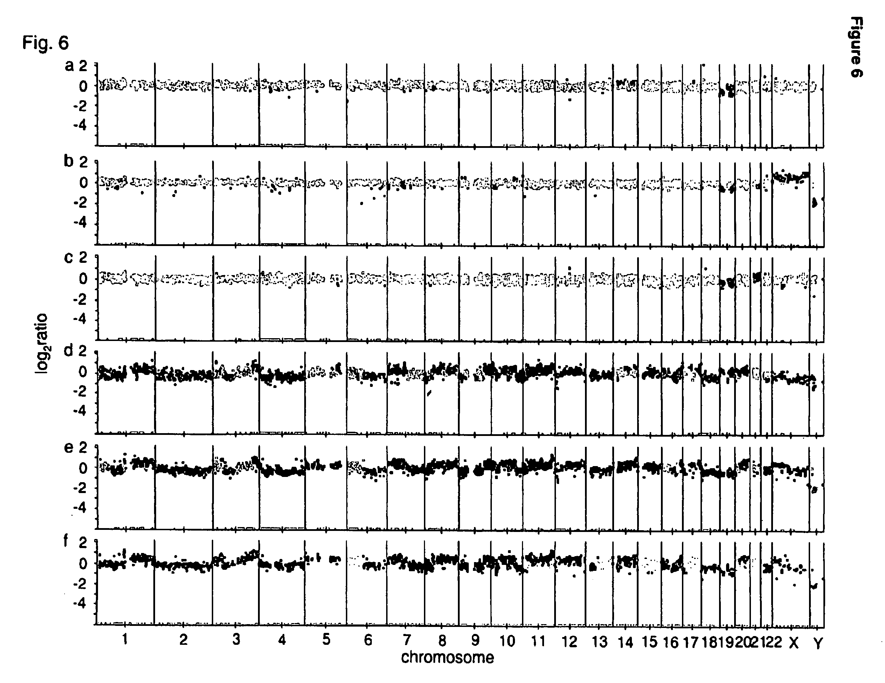

Figure 6. Hybridization of five different adapter-linker amplified single cell DNA samples versus male reference DNA on 3K single cell BAC array.
   **a**, normal male single cell, **b**, normal female single cell, **c**, single PBL from patient with trisomy 21 (male), **e** and **f**, single cells from T47D cell line. **d**, hybridization of genomic DNA obtained from T47D cell line using a standard-protocol. Note the high degree of similarity when comparing single cell arrayCGH profiles and the genomic DNA of T47D hybridization. Note also the small differences between the two single cells and the genomic pooled DNA, caused by enhanced cellular resolution. The log₂ transformed test to reference DNA fluorescence intensities ratios are plotted against the chromosomal locations of the BAC clones. Very light grey color of dots indicates normal clones, light shaded grey indicates loss and dark shaded grey indicates gain of chromosomal material. Black indicates high-copy amplifications. (data normalization and visualization was performed with CAPweb software).

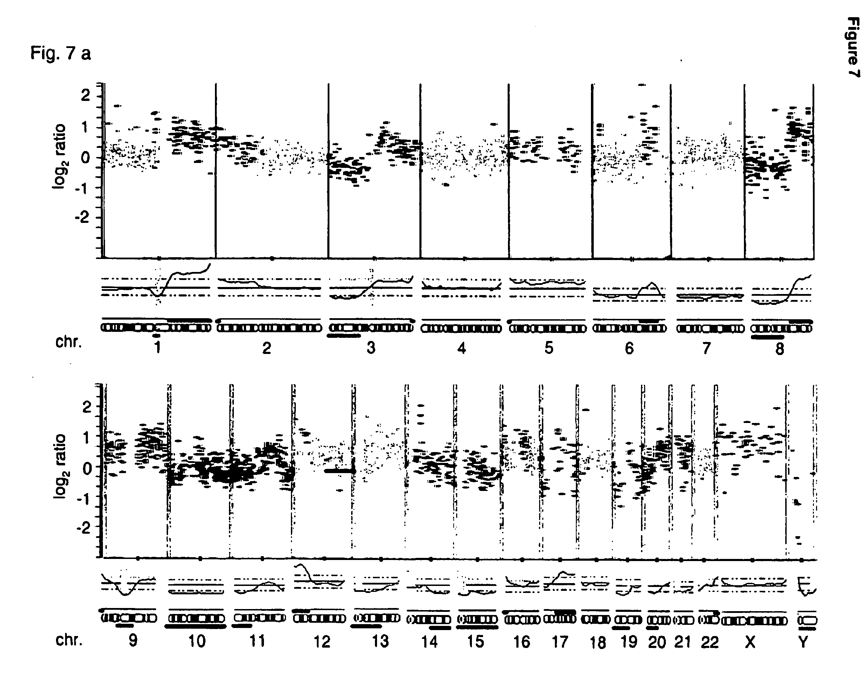

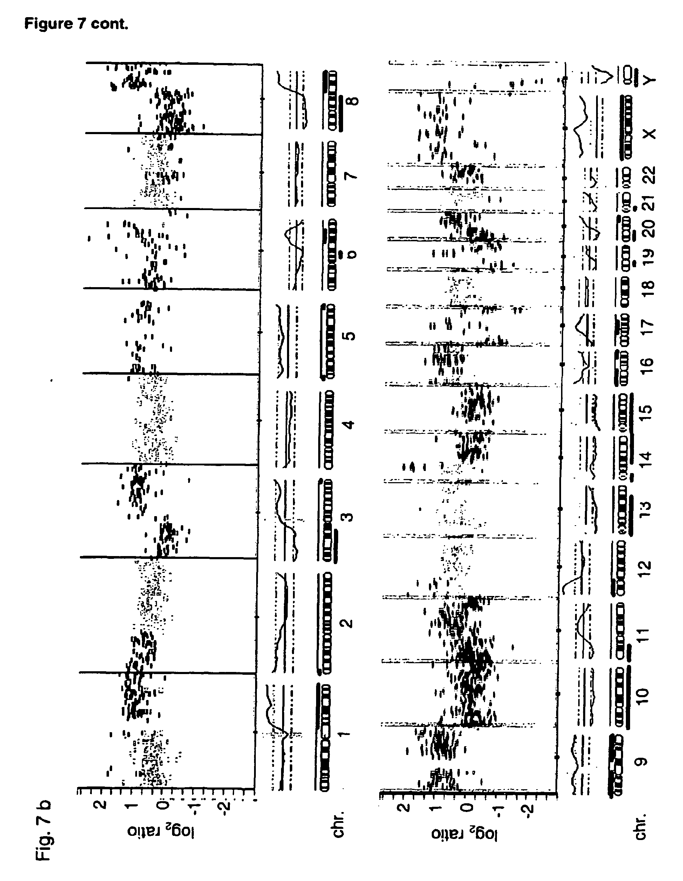

Figure 7. Hybridization of two different adapter-linker amplified single tumor cell DNA samples.
   These samples were generated from cells isolated from the bone marrow of one breast cancer patient, versus male reference DNA on 3K single cell BAC array. The metaphase CGH profile obtained by hybridizing the same DNA is plotted beneath the arrayCGH profile. Chromosome ideograms are placed horizontally, in a way that the genomic position on the metaphase and arrayCGH plot correspond to each another and enables a direct comparison **a**, single tumor cell 1, **b,** single tumor cell 2. Note the high correlation of arrayCGH and metaphase CGH profiles for both single tumor cells. Note also that more and smaller genomic imbalances are detected with the arrayCGH. For example 2 small inserted deletions on the q-arm of chromosome 11 (7 Mb and 500 kb in size; going from centromer to telomer) and the high-copy amplification on chromosome 14p (2.7 Mb) detectable as conserved imbalances in both single cells. The log₂ transformed test to reference DNA fluorescence intensities ratios are plotted against the chromosomal locations of the BAC clones. Very light grey color of dots indicates normal clones, light shaded grey indicates loss and dark shaded grey indicates gain of chromosomal material. Black indicates high-copy amplifications. (data normalization and visualization was performed with CAPweb software).

### Examle 1

### General methods

### (A) BAC isolation

BAC clones were grown in LB medium containing 20 µg/ml chloramphenicol. Standard preparation of BAC-DNA was performed by Qiagen midi columns, according to the manufacturer's recommendations. For preparation of BAC-DNA by pulsed-field electropheresis, bacterial agarose-inserts were prepared as described with minor modifications¹² and digested with PI-Scel. Briefly, the bacteria were pelleted by centrifugation, embedded in 1% low melt agarose and lysed with lysozym (over night incubation at 37°C with 6 mM Tris-HCl pH 7.5, 1 M NaCl, 100 mM EDTA, 0.5% Brij-58, 0.2% deoxycholate, 0.5% lauryl sarkosine, 30 U/ml RNase (DNase free), 50.000 U/ml lysozym) followed by proteinase K digestion of the cellular proteins (over night incubation at 50°C with 0.5 M EDTA pH 9.0, 1% lauroyl-sarcosine, 1.5 U/ml proteinase K). Fragmented DNA was removed by gel-electrophoresis at 80 V for 2 h in 0.5 x TAE buffer. The BAC-DNA was digested with the restriction enzyme PI-Scel for 3 h at 37°C after giving the enzyme time to diffuse into the agarose-insert overnight on ice (1.5 U/100 µl PI-Scel; 100 mM KCI; 10 mM Tris-HCl pH 8.6; 10 mM MgCl₂; 1 mM dithiothreitol; 3.5 µg BSA). Subsequently, the proteinase K digest was repeated. After inactivation of proteinase K by auto-hydrolysis, the agarose-inserts were equilibrated in TE, loaded on a 1% low melt agarose gel, and the DNA was separated by PFGE (Amersham Bioscience) according to manufacturers recommendations in 0.5 x TAE. The BAC-DNA was gel-extracted by an over night agarase digest (1 Unit agarase per 100 µl gel-volume in 30 mM Bis-Tris pH 4.5; 10 mM EDTA) and precipitated with isopropanol and Na-acetate (3 M) at 4°C. After washing with 70% ethanol, the DNA was resuspended in 50 µl TE.

### (B) Amplification of isolated DNA

All BAC-DNA preparations were amplified by the adapter-linker method with minor modifications^{7,19} and by DOP-PCR as described²⁰. To avoid cross-hybridization with the adaptors from the single cell PCR products, different sequences of the adaptors were selected that were ligated to BAC DNA, i.e. 5'-CTGTGTCTGACGACTCAGTCT-3' and 5'-TAAGACTGAGTCdd-3'. Phi29 amplifications were performed with the TempliPhi Kit (Amersham Biosciences), according to manufacturer's recommendations.

### (C) Preparation of microarray

BAC probes were ethanol precipitated after PCR or rolling circle amplification and DNA concentration was adjusted to 0.4 g/µl in 3x SSC. Probes were applied in replicas of five to amino-silane coated slides (Corning, GAPS II) using an Omnigrid 100 spotting robot (Genomic Solutions, Ann Arbor, U.S.A.). DNA was immobilized by UV cross-linking followed by 30 min baking at 80°C.

### (D) Labeling and hybridization

Adapter-linker amplified target and reference DNA were labeled by PCR in presence of Cy3 or Cy5 conjugated nucleotides, respectively. The 100 µl PCR mix comprised 13 mM Tris-HCI, pH 9.5, 3.25 mM MgCl₂, 5 µM LIB1 primer¹⁹, 200 µM dATP and dGTP, 180µM dTTP and dCTP, 20µM Cy3/Cy5-dUTP (Amersham Biosciences) and Cy3/5-dCTP (Amersham Biosciences), 16 U ThermoSequenase (Amersham Biosciences), and 1 µl template DNA). The MJ-research thermocycler was programmed to nine cycles of 94°C 1 min, 60°C 30 sec, ramp to 72°C with 0.2°C/sec, 72°C 5 min with 60 sec extension/cycle. After labeling, PCR adaptors were removed by Trul digestion (100 U Trul (Fermentas) and 10 µl of 10 x buffer containing 70 mM MgCl₂, 1 M KCl and 1 mg/ml BSA). To remove unincorporated dye, DNA was applied to a Sephadex G50 column. Eluted labeled DNA was precipitated together with 80 µg C₀t-1-DNA (Roche) and resolved in 50 µl master-mix (50% formamide, 2x SSC, 4% SDS and 0.1 g/ml dextrane sulphate (MW>500000; Fisher Biotech). Hybridization was done without cover slip at 42°C in a humidified chamber for two nights on a shaker as described⁴. Before hybridization of target DNA, the microarray was pre-hybridized over night at 42°C with 50 µl master-mix as above containing 250 µg Hering sperm DNA, 10 µg sonicated *E. coli* genomic DNA and 100 µg mock control DNA (adapter-linker PCR contamination product, amplified without addition of cellular DNA).

### (E) Single-cell PCR and Conventional-CGH

Single cell adapter-linker PCR and conventional-CGH was performed as published^{2,7,19}.

### (F) Statistical analysis of array data

Data analysis was performed according to Quackenbush²¹. In brief, the geometric means of the BAC clone replicas were calculated and the fluorescence signal intensities of the Cy3 and Cy5 channel were globally normalized. BAC clones in chromosomal areas affected by copy number losses or gains were excluded when the signal intensity was normalized. For all BAC clones, the ratio of the normalized fluorescence intensity was log₂ transformed and plotted against the chromosomal location. Copy number differences were assumed to be significant, if the signal intensity ratios of the affected BAC clones exceeded the upper or lower thresholds defined by the three-times standard deviation that was calculated from the baseline noise.

### (G) qPCR

Quantitative PCR was performed using the LightCycler System (Roche) and the LightCycler FastStart DNA Master SYBR Green I kit (Roche), according to the manufacturer's recommendation. The forward and reverse primer sequences for the amplification of *E. coli TRPE* genomic sequence and the pBACe3.6 vector sequence were taken from Foreman et.al.¹¹, without the additional probe beacon. The crossing points of the samples that represent the threshold PCR cycle at which the exponential growth of the fluorescence signal was initially detected, were determined by the LightCycler Relative Quantification Software (Roche). The total amounts of vector and *E. coli* DNA in the samples were calculated in an exponential equation with the amplification efficacy of the vector or *E. coli* PCR reactions (1.92 and 1.93, respectively) as base to the power of the crossing points for each sample pair. The ratio of vector versus *E. coli* DNA was determined and expressed as percentage of contaminating *E. coli* DNA in the samples.

### Example 2

### Microarray CGH enabling the detection of DNA-copy number imbalances of single cells

First arrays were tested that had been successfully used to analyze large amounts of DNA from primary tumors⁹. By hybridizing DNA amplified from single cells of known karyotypes onto these arrays, however, the chromosome copy numbers of the test samples could not be correctly determined (data not shown). It was reasoned that bacterial or plasmid DNA contaminating the single cell preparations hybridizes to bacterial or plasmid DNA present on the BAC array causing large signal fluctuations.

Such contaminating DNA could be introduced during sample preparation with the enzymes that are needed for the amplification of cellular DNA (proteinase K, Mse I, T4 DNA ligase and Taq polymerase). In standard chromosomal CGH contaminating DNA from bacterial sources would not hybridize to human metaphases. This hypothesis was tested by hybridizing mock control samples that had been prepared without the addition of cellular DNA. Indeed, no signals were detected on metaphase spreads while the intensely fluorescent hybridization pattern on BAC arrays could hardly be distinguished from samples containing DNA amplified from human cells (data not shown).

Previously it was noted that non-human DNA on BAC arrays interferes with exact measurements in hybridization experiments¹⁰ and that the purity of plasmids containing the human DNA depends on how the BAC preparation is performed¹¹. To reduce the amount of non-human DNA in the BAC preparations various approaches were tested, including long-range PCR, *in vitro* transcription of the BAC inserts, subtractive enrichment of the BAC-DNA, oligo-nucleotide based arrays and the purification of the BAC-clones by pulsed field gel electrophoresis (PFGE)¹². The last method was clearly superior to all other strategies, because it enabled both highest recovery and best purification of the BAC DNA. The RP-11 BAC clones used for this purpose contain a recognition site of the homing endonuclease PI-Scel in the pBACe3.6 vector sequence which is not present in the *E. coli* genome¹³. Therefore, the exclusive linearization of the RP-11 BAC clones enables the physical separation from the *E. coli* genomic DNA during the gel run (Figure 1), whereas circular BACs could not be separated under the applied conditions. The purification of the BAC DNA from *E. coli* DNA was verified by quantitative (q) PCR using primers for the *E. coli* TRPE gene¹¹. While only traces of contaminating *E. coli* DNA were detected in PFGE-purified samples (0.32% ± 0.11 SD), BAC clones conventionally prepared using Qiagen columns were contaminated to a varying degree (1.3-25.2%, average 8.0% ± 6.0 SD) (Figure 2). As the average purity of these Qiagen BAC preparations was similar to reported data (6-10%)¹¹, they apparently represent those spotted on most of the current BAC arrays.

The DNA from 30 RP-11 clones (Table 1), either isolated by PFGE or by conventional Qiagen columns, was used for the generation of a microarray. The two DNA preparations of a BAC clone were amplified using adapter-linker-PCR, DOP-PCR and Phi29 rolling circle amplification, resulting in six different DNA samples obtained from each RP-11 clone. The applied amplification methods had been previously described for array preparation after conventional BAC preparation^{6,10,14,15}. All DNA preparations of each BAC clone were spotted onto one glass slide (five replicas each) for a direct comparison of the resulting hybridization signals for each labeled DNA sample.

First, 500 ng unamplified DNA extracted from peripheral blood cells from a patient with trisomy 21 were hybridized to the array. All BAC clones from chromosome 21 displayed the expected copy number gain regardless which probe preparation and amplification method was used (Figure 2 a-f), verifying the suitability of both PFGE and conventional BAC DNA isolation methods for this application. However, when DNA from a single leukocyte of the same patient was amplified by adapter-linker PCR⁷ and subsequently hybridized onto the differently prepared BAC clone sets, the trisomy 21 was only detected by PFGE purified clones (not shown; see below). Array-CGH with DNA from single leukocytes of healthy donors (n=3) showed normal ratios for all BAC clones (data not shown). When the effects of amplification methods on the ratios detected by PFGE isolated clones were compared, the reliability of measurements by adaptor-linker amplified DNA was superior to DOP-amplified DNA and Phi29-amplified DNA. This might be due to matched DNA fragments in probe and target, because both were generated by Msel digest. In addition, the hybridization noise originating from contaminating sequences was blocked by pre-hybridizing the array with unlabeled blocking DNA. As blocking DNA the mock control of the respective single cell amplification experiment was used, because it presumably contains similar contaminating DNA and is therefore best suited to increase signal to noise ratios. Interestingly, the blocking DNA improved only the quality of hybridization with the PFGE-purified samples on the array (Figure 3 a-c), while little or no effect could be observed for the conventional BAC preparations (Figure 3 d-f). Thus, the blocking of contaminating DNA before or during hybridization is successful only as long as the contamination does not exceed a certain threshold.

Finally, single disseminated tumor cells and DNA from formalin-fixed paraffin-embedded tissue isolated by laser microdissection were analysed. To this end tumor samples were selected for which previously chromosomal losses or gains by classical CGH within those regions that were covered by our BAC clones had been determined^{2,8}. For the single cells the noise on any of the conventionally prepared BAC arrays prohibited an analysis while on the PFGE-purified arrays both cells could be evaluated. The first single tumor cell (TC-1) displayed a deletion of chromosome 17 and the second (TC-2) a gain of chromosome 5 - as expected from the metaphase CGH profile (Figure 4 a and b). Similar results were obtained using formalin-fixed and paraffin-embedded tissues after microdissection and amplification. As before, gains and losses predicted by metaphase CGH on chromosome 17 were detected by microarray analysis on PFGE-purified and adaptor-linker-amplified DNA (Figure 4 c), while on BAC clones prepared by the standard BAC protocols only the high copy amplification of the *HER2* gene on chromosome 17q21.2 could be detected.

A second generation of microarrays was constructed using a total of 94 RP11-BAC clones (Table 2) in order to verify reproducibility of the method with a different clone-set, independent amplification and spotting. Preparation and PCR-amplification of BAC-DNA was performed as described above. For spotting of samples, DNA was resolved in water following precipitation and adjusted to a concentration of 0.8 µg/µl. Addition of 2x concentrated *sciSPOT* spotting buffer for oligos (Scienion) diluted DNA to a final spotting concentration of 0.4µg/µl. After letting the slides dry, an aftertreatment was performed by the manufacturer with *sciPROCESS* buffer. Hereby slides were washed in 55°C *sciPROCESS* buffer for 15 minutes, then washed once in 55°C distilled water for 5 minutes and twice in 25°C distilled water for 25 minutes. Then the slides were dried by centrifugation at 900rpm. Spot quality was assessed by Sybr Green dyeing.

It proved that good spot quality and morphology were dependent on spotting parameters, such as the spotting buffer, where ethylene glycol and DMSO produced spots that were not suited for hybridization, since DNA did not adhere to the slide surface. Scienion Oligo-spotting buffer finally turned out to be superior to all tested spotting buffers, yielding homogeneous spots. Additionally the procedure of PCR-cleanup played an important role, Microcon-column purification being inferior to ethanol-precipitation. Informative hybridization results could additionally only be achieved when BAC-DNA was spotted on GAPS II slides, whereas on CodeLink and Epoxy-slides hybridization results where of low quality.

It was found that choice of reference DNA has a great impact on the hybridization. It was tested whether normal single cells yield better hybridization results than pools of 10, 100 or 1000 cells when cohybridized with sample DNA, due to the fact, that the amount of contamination in reference and probe would be comparable. Unexpectedly though, pools of 1000 cells were the optimal reference DNAs.

Incorporating these findings, a second generation chip could be generated allowing to reproduce results observed with the first generation chip, shown in Figure 5a and b. Hybridization of single tumor cell displaying deletion of chromosome 17 (TC-1) is shown in Figure 5a. Normal single cell DNA hybridized without detectable amplifications or deletions (Figure 5b).

### Example 3

### New generation of microarrays

A third generation of microarrays was constructed (3K array) containing a total of 2940 RP11-BAC clones, so that an average resolution of 1 Mb throughout the whole human genome was achieved. The preparation and PCR-amplification were performed as described above for the 30 BAC clone array. Spotting was performed in 1 x *sciSPOT* spotting buffer with a final DNA concentration of 0.4 µg/µl. Labelling of probes as well as hybridization and washing were performed as described above. Data analysis was performed with CAPweb software (freely available at http://bioinfo-out.curie.fr/CAPweb/) and visualization was perfomed using VAMP. Only features with reference fluorescence intensity at least 2 fold over the background were taken into account. For all other parameters, default values of the software were used.

Figure 6 a-c and e-f show hybridization of globally amplified DNA from single cells onto the 3K array. Panel and b show hybridization of two normal single cells, where throughout the whole array normal copy numbers are measured for all autosomal clones (except chromosome 19 which is not included into further analysis). In figure 6b the female gender of the probe is easily detected in the gonosomal clones. Figure 6c shows an hybridization with the same single PBL from a patient with trisomy 21 as the one shown in fig. 3. The gain of genomic material on chromosome 21 is easily detected, the other clones measure a normal karyotype. Figure 6d shows the hybridization profile of genomic DNA derived from T47D cell line according to standard protocols. In e and f single cell PCR products of the same cell line are hybridized and show a high degree of similarity but also subtle variances, due to the enhanced cellular resolution achieved by single cell array CGH.
In figure 7 a and b hybridization of single cell PCR-products of two tumor cells derived from the bone marrow of one breast cancer patient is shown in comparison to the hybridization profile generated by conventional single cell metaphase CGH. A high degree of similarity can be observed. The array CGH confers gain of information due to enhanced spatial resolution compared to metaphase CGH as demonstrated by the detection of two small inserted deletions on the q-arm of chromosome 11 (7 Mb and 500 kb of size; going from centromer to telomer) and the high-copy amplification on chromosome 14p (2.7 Mb of size) detectable as conserved imbalances in both single cells.

**Table 1**

| BAC clone | Chromosome | Map position | FISH | Accession |
|---|---|---|---|---|
| RP11-103I11 | 5 | 3,875 K | 5p15.33 | AC092319.2 |
| RP11-260e18 | 5 | 16,702 K | 5p15.2 | AC024588.4 |
| RP11-5n11 | 5 | 31,730 K | 5p13.3-5p14 | AC022447.6 |
| RP11-252i13 | 5 | 107,088 K | 5q21 | AC024587.5 |
| RP11-21c10 | 5 | 134,022 K | 5q31.1-5q31.2 | AC 106763.2 |
| RP11-86c20 | 5 | 152,699 K | 5q33 | AC091991.2 |
| RP11-170113 | 5 | 155,253 K | 5q33.2 | AC114959.2 |
| RP11-6219 | 15 | 37,310 K | 15q14-15q15 | AC027386.3 |
| RP11-99118 | 15 | 57,379 K | 15q21-15q22 | AC091748.3 |
| RP11-2i17 | 15 | 70,146 K | 15q23 | AC020779.1 0 |
| RP11-106m3 | 15 | 70,319 K | 15q23 | AC009690.1 7 |
| RP11-57p19 | 15 | 92,187 K | 15q26.2 | AC009432.7 |
| RP11-26n16 | 17 | 992 K | 17p13.3 | B86973 |
| RP11-135n5 | 17 | 2,655 K | 17p13.2 | AC015799.2 3 |
| RP11-89a15 | 17 | 8,624 K | 17p12 | AC135070.2 |
| RP11-55c13 | 17 | 8,662 K | 17p12-17p13 | AC116169.5 |
| RP11-100112 | 17 | 28,824 K | 17q11.2 | AQ345666 |
| RP11-73f15 | 17 | 29,287 K | 17q11.2 | AQ266525 |
| RP11-268h17 | 17 | 39,404 K | 17q21.2 | AQ490954 |
| RP11-29c11 | 17 | 40,020 K | 17q21.1-17q21.2 | B87496 |
| RP11-266i24 | 17 | 41,501 K | 17q21 | AQ490757 |
| RP11-110h20 | 17 | 47,416 K | 17q21.3 | AQ320062 |
| RP11-52b5 | 17 | 65,437 K | 17q24 | AC027772.2 |
| RP11-160f4 | 17 | 79,067 K | 17q25.2 | AQ377826.1 |
| RP11-108h5 | 21 | 26,825 K | 21q21 | AQ348670 |
| RP11-74d19 | 21 | 28,634 K | 21q21 | AQ266734 |
| RP11-32a2 | 21 | 29,862 K | 21q21.2- | AP000239.2 |
| | | | 21q21.3 | |
| RP11-96h21 | 21 | 30,958 K | 21q22.2 | AQ314195 |
| RP11-14b21 | 21 | 31,285 K | 21q22.1 | AP000245.3 |
| RP11-114h1 | 21 | 41,228 K | 21q22.2-21 q22.3 | AQ383555 |

**Table 2**

| BAC clone | Chromosome | Map position | FISH | Marker |
|---|---|---|---|---|
| RP11-94d19 | 3 | 60.8 Mb | 3p14 | AC016965 |
| RP11-108a8 | 3 | 63.3 Mb | 3p14 | AC072020 |
| RP11-180g14 | 3 | 59.5 Mb | 3p14 | AFMA072ZH |
| RP11-114p15 | 3 | 62.6 Mb | 3p14 | AC048336 |
| RP11-229a12 | 3 | 57.5 Mb | 3p14.3(FHCRC) | AC016923 |
| RP11-111p21 | 3 | 45.4 Mb | 3p21.2 | AC063916 |
| RP11-189h19 | 3 | 44.7 Mb | 3p21.2 | AC069071 |
| RP11-122d19 | 3 | 54.1 Mb | 3p21.2 | AC018354 |
| RP11-169g24 | 3 | 56.3 Mb | 3p21.2 | AC018920 |
| RP11-3911 | 3 | 55.8 Mb | 3p21.2 | AQ029524 |
| RP11-120c2 | 3 | 57.4 Mb | 3p21.2 | AC069438 |
| RP11-121d3 | 3 | 14.4 Mb | 3p21-22 | AC016963 |
| RP11-81N11 | 4 | 34.7 Mb | 4p13 | AQ281893 |
| RP11-81H11 | 4 | 31.7 Mb | 4p15.1 | AQ281616 |
| RP11-81N5 | 4 | 13.1 Mb | 4p15.3 | AQ281881 |
| RP11-81L15 | 4 | 14.7 Mb | 4p15.3 | AQ281853 |
| RP11-80H22 | 4 | 109.9 Mb | 4q25-4q26 | AQ284104 |
| RP11-81J9 | 4 | 110.0 Mb | 4q26 | AQ281666 |
| RP11-80P12 | 4 | 132.5 Mb | 4q28 | AQ284210 |
| RP11-48F12 | 4 | 22.1 Mb | 4q32.3 | AQ200675 |
| RP11-181G18 | 4 | 16.7 Mb | 4q35/21q11.2 | SHGC-21147 |
| RP11-9G14 | 5 | 41.3 Mb | 5p12 | AFM276VB1 |
| RP11-5N11 | 5 | 31.7 Mb | 5p13.3-5p14 | AC022447 |
| RP11-67P13 | 5 | 32.1 Mb | 5p13-5p14 | AQ203092 |
| RP11-19119 | 5 | 58.9 Mb | 5q11.2g-5q11.2 | A0003242 |
| RP11-15P2 | 5 | 66.3 Mb | 5q12 | AC063978 |
| RP11-47N20 | 5 | 81.4 Mb | 5q13-5q14 | AQ194964 |
| RP11-252113 | 5 | 107.0 Mb | 5q21 | AC024587 |
| RP11-115L24 | 5 | 103.2 Mb | 5q21 | AC009815 |
| RP 11-81 C5 | 5 | 115.3 Mb | 5q22-5q23 | AQ284841 |
| RP11-15P5 | 5 | 120.4 Mb | 5q23.1 | B75415 |
| RP11-86C20 | 5 | 152.7 Mb | 5q33 | AC091991 |
| RP11-39C2 | 6 | 47.1 Mb | 6p12 | AL355518 |
| RP11-7912 | 6 | 43.5 Mb | 6p21.1-6p21.2 | AQ283086 |
| RP11-79J17 | 6 | 33.0 Mb | 6p21.2-6p21.3 | AQ284306 |
| RP11-78P9 | 6 | 108.6 Mb | 6q21 | AFMA309X |
| RP11-28K19 | 6 | 119.6 Mb | 6q22.2-6q22.3 | B87366 |
| RP11-18B17 | 15 | 58.6 Mb | 15q21.3 | SHGC-11862 |
| RP11-30I7 | 15 | 54.3 Mb | 15q21.3 | B85180 |
| RP11-99L18 | 15 | 57.4 Mb | 15q21-15q22 | AC091748 |
| RP11-120011 | 15 | 57.7 Mb | 15q22.1 | A0349760 |
| RP11-13J23 | 15 | 58.7 Mb | 15q22.2 | B83398 |
| RP11-64K10 | 15 | 68.7 Mb | 15q23 | AC010076 |
| RP11-116L7 | 15 | 83.8 Mb | 15q25 | AQ345509 |
| RP11-68P22 | 15 | 83.0 Mb | 15q25 | AQ241268 |
| RP11-55C13 | 17 | 8.4 Mb | 17p12-17p13 | AC116169 |
| RP11-135N5 | 17 | 2.4 Mb | 17p13.2 | AC015799 |
| RP11-26N16 | 17 | 922.2 Kb | 17p13.3 | B86972 |
| RP11-73F15 | 17 | 26.2 Mb | 17q11.2 | AQ266525 |
| RP11-252N11 | 17 | 30.4 Mb | 17q12 | AQ504453 |
| RP11-58O8 | 17 | 31.0 Mb | 17q12 | AQ 199256 |
| RP11-50F16 | 17 | 55.6 Mb | 17q12/17q23 | AQ053427 |
| RP11-266I24 | 17 | 38.3 Mb | 17q21 | AQ490753 |
| RP11-29C11 | 17 | 36.8 Mb | 17q21.1-17q21.2 | B87495 |
| RP11-241H2 | 17 | 39.0 Mb | 17q21.1-17q21.2 | AQ482539 |
| RP11-268H17 | 17 | 36.4 Mb | 17q21.2 | AQ490951 |
| RP11-42M14 | 17 | 48.1 Mb | 17q22 | AQ046621 |
| RP11-118K23 | 17 | 55.1 Mb | 17q23 | SHGC-36454 |
| RP11-52B5 | 17 | 62.4 Mb | 17q24 | AC027772 |
| RP11-76G4 | 17 | 70.8 Mb . | 17q25.1 | AQ265611 |
| RP11-160F4 | 17 | 76.0 Mb | 17q25.2 | AQ377826 |
| RP11-84J3 | 17 | 74.6 Mb | 17q25.2-17q25.3 | SHGC-11286 |
| RP11-26B13 | 17 | 74.1 Mb | 17q25.2-17q25.3 | B86818 |
| RP11-88I12 | 19 | 19.0 Mb | 19p12-19p13.1 | AFMA123XF |
| RP11-84C17 | 19 | 7.8 Mb | 19p13.3 | AFMB299W |
| RP11-46I12 | 19 | 34.4 Mb | 19q12-19q13.1 | AQ201106 |
| RP11-35J17 | 19 | 58.8 Mb | 19q13.4 | AQ045816 |
| RP11-48M7 | 20 | 3.8 Mb | 20p12 | AQ198007 |
| RP11-9G21 | 20 | 6.1 Mb | 20p12.3 | AFM317TE5 |
| RP11-149O7 | 20 | 5.5 Mb | 20p12-20p13 | AQ377575 |
| RP11-153M12 | 20 | 989.4 Kb | 20p13 | AQ387835 |
| RP11-235C20 | 20 | 5.1 Mb | 20p13 | AFMB352X D |
| RP11-78P4 | 20 | 2.0 Mb | 20p13 | AQ281462 |
| RP11-24E15 | 20 | 467.4 Kb | 20p13 | B86599 |
| RP11-26F18 | 20 | 3.2 Mb | 20p13 | AC016217 |
| RP11-107L14 | 20 | 2.6 Mb | 20p13 | AQ316350 |
| RP11-61A21 | 21 | 15.4 Mb | 21q11.1 | AQ199973 |
| RP11-31B6 | 21 | 15.1 Mb | 21q11.2-21q21 | AQ046029 |
| RP11-106K13 | 21 | 15.2 Mb | 21q11.2-21q21 | AQ315211 |
| RP11-143A3 | 21 | 18.2 Mb | 21q11.2-21q21 | SHGC-2319 |
| RP11-86I21 | 21 | | 21q21 | AQ283088 |
| RP11-53K11 | 21 | 25.8 Mb | 21q21 | AQ051878 |
| RP11-49J9 | 21 | 21.1 Mb | 21q21.1 | AQ053086 |
| RP11-617 | 21 | 26.9 Mb | 21q21.2-21q21.3 | B48248 |
| RP11-83L17 | 21 | 26.9 Mb | 21q21.3 | AFM263XF5 |
| RP11-276A17 | 21 | 31.4 Mb | 21q21.3 | AFM288YG5 |
| RP11-147H1 | 21 | 32.3 Mb | 21q21.3 | AQ373665 |
| RP11-125H1 | 21 | 31.4 Mb | 21q21.3 | AQ348941 |
| RP11-30N6 | 21 | 29.8 Mb | 21q21.3 | B85241 |
| RP11-115H17 | 21 | 27.1 Mb | 21q21.3-21q22.1 | AQ346443 |
| RP11-12M14 | 21 | 31.3 Mb | 21q21-21q22.1 | B75909 |
| RP11-191I6 | 21 | 31.5 Mb | 21q21-21q22.1 | AFM288YG5 |
| RP11-17020 | 21 | 35.3 Mb | 21q22.1 | B82827 |
| RP11-54F16 | 21 | 32.8 Mb | 21 q22.1 | AQ082133 |

### References

1. Pardal, R., Clarke, M.F. & Morrison, S.J. Applying the principles of stem-cell biology to cancer. Nat Rev Cancer 3, 895-902. (2003).
2. Schmidt-Kittler, O. et al. From latent disseminated cells to overt metastasis: genetic analysis of systemic breast cancer progression. Proc Natl Acad Sci U S A 100, 7737-42 (2003).
3. Braun, S. et al. Cytokeratin-positive cells in the bone marrow and survival of patients with stage I, II, or III breast cancer. N Engl J Med 342, 525-33. (2000).
4. Pinkel, D. et al. High resolution analysis of DNA copy number variation using comparative genomic hybridization to microarrays. Nat Genet 20, 207-11 (1998).
5. Solinas-Toldo, S. et al. Matrix-based comparative genomic hybridization: biochips to screen for genomic imbalances. Genes Chromosomes Cancer 20, 399-407 (1997).
6. Ishkanian, A.S. et al. A tiling resolution DNA microarray with complete coverage of the human genome. Nat Genet 36, 299-303. Epub 2004 Feb 15. (2004).
7. Klein, C.A. et al. Comparative genomic hybridization, loss of heterozygosity, and DNA sequence analysis of single cells. Proc Natl Acad Sci U S A 96, 4494-9. (1999).
8. Stoecklein, N.H. et al. SCOMP is superior to degenerated oligonucleotide primed-polymerase chain reaction for global amplification of minute amounts of DNA from microdissected archival tissue samples. Am J Pathol 161, 43-51. (2002).
9. Fritz, B. et al. Microarray-based copy number and expression profiling in dedifferentiated and pleomorphic liposarcoma. Cancer Res 62, 2993-8. (2002).
10. Fiegler, H. et al. DNA microarrays for comparative genomic hybridization based on DOP-PCR amplification of BAC and PAC clones. Genes Chromosomes Cancer 36, 361-74 (2003).
11. Foreman, P.K. & Davis, R.W. Real-time PCR-based method for assaying the purity of bacterial artificial chromosome preparations. Biotechniques 29, 410-2 (2000).
12. Smith, C.L. & Cantor, C.R. Preparation and manipulation of large DNA molecules: advances and applications. Trends in Biochemical Sciences 12, 284-287 (1987).
13. Frengen, E. et al. A modular, positive selection bacterial artificial chromosome vector with multiple cloning sites. Genomics 58, 250-3 (1999).
14. Lage, J.M. et al. Whole genome analysis of genetic alterations in small DNA samples using hyperbranched strand displacement amplification and array-CGH. Genome Res 13, 294-307. (2003).
15. Snijders, A.M. et al. Assembly of microarrays for genome-wide measurement of DNA copy number. Nat Genet 29, 263-4 (2001).
16. Klein, C.A. The systemic progression of human cancer: A focus on the individual disseminated cancer cell - the unit of selection. Adv Cancer Res 89, 35-67 (2003).
17. Hasty, P., Campisi, J., Hoeijmakers, J., van Steeg, H. & Vijg, J. Aging and genome maintenance: lessons from the mouse? Science 299, 1355-9. (2003).
18. Lengauer, C., Kinzler, K.W. & Vogelstein, B. Genetic instability in colorectal cancers. Nature 386, 623-627 (1997).
19. Klein, C.A. et al. Genetic heterogeneity of single disseminated tumour cells in minimal residual cancer. Lancet 360, 683-9 (2002).
20. Telenius, H. et al. Degenerate oligonucleotide-primed PCR: general amplification of target DNA by a single degenerate primer. Genomics 13, 718-25. (1992).
21. Quackenbush, J. Microarray data normalization and transformation. Nat Genet 32, 496-501. (2002).
22. US-Patent No. 6,210,878 B1 (2001).
23. Solinas-Toldo S. et al. Matrix-based comparative genomic hybridization: biochips to screen for genomic imbalances. Genes Chromosomes Cancer 20(4), 399-407. (1997).
24. EP-B1 1 109 938
25. US-Patent No. 6,197,501 B1 (2001).
26. Osolgawa, K. et al., Genome Research 11 :483-496 (2001).
27. Current Protocols in Molecular Biology, Vol. 1, 5.9.1, Supplement 55, "Consruction of Bacterial Artificial Chromosome (BAC/PAC) Libraries".

## Claims

1. A method for isolating artificial chromosomes from a library of artificial chromosomes which are free of host DNA, said method comprising the following steps:
(a) preparing a library of artificial chromosomes with digested chromosomal DNA by use of a vector containing a recognition site for a restriction enzyme (x) which is not present within the genome of the host used for propagation of the artificial chromosomes;
(b) digesting the DNAs prepared from the library of artificial chromosomes of step (a) with restriction enzyme (x) after propagation of the library; and
(c) separating the digested linear artificial chromosomes of the library from the undigested circular host DNA by use of a separation method allowing to distinguish between linear and circular DNA.

2. The method of claim 1. wherein said library of artificial chromosomes is a library of BAC clones.

3. The method of claim 1 or 2, wherein said vector contains a single recognition site for the restriction enzyme (x).

4. The method of any one of claims 1 to 3, wherein said restriction enzyme (x) is PI-Scel.

5. The method of any one of claims 1 to 4, wherein said host is *E.coli.*

6. The method of claim 5, wherein said chromosomal DNA is obtained from cells of a tissue sample.

7. The method of any one of claims 1 to 6, wherein said chromosomal DNA is obtained from human cells.

8. The method of any one of claims 1 to 7, wherein said method for separating the digested linear artificial chromosomes from the undigested circular host DNA is Pulsed-Field-Gel-Electrophoresis (PFGE).

9. The method of any one of claims 1 to 8, wherein in step (a) the chromosomal DNA is isolated from cells which have no known numerical changes or imbalances in their DNA.

10. Use of artificial chromosomes obtainable according to a method of any one of claims 1 to 9 in a method for DNA analysis of a sample, wherein said method for DNA analysis comprises the following steps:
(a) preparing a microarray with the artificial chromosomes;
(b) hybridizing DNA obtained from (a) target cell(s) with the artificial chromosomes of said microarray; and
(c) evaluating the hybridization results,
wherein prior to step (b) the target DNA has been amplified, wherein said DNA has been obtained from a small cell population or a single cell and wherein steps (b) and (c) are performed as "Comparative Genomic Hybridization" (CGH).

11. The use according to claim 10, wherein said target DNA has been amplified by adapter-linker PCR.

12. The use according to claim 10 or 11, wherein said method for DNA analysis is a method for detecting numerical changes and/or imbalances in DNA of a sample, wherein said target cells are cells suspected to have numerical changes and/or imbalances in their DNA.

13. The use according to any one of claims 10 to 12 for the study of minute amounts of ex *vivo* DNA from premalignant lesions or very small tumors, from biopsies before and after systemic therapy, archived tumor samples of patients with known clinical outcome, analysis of single metastastic precursor cells or of circulating fetal cells in the maternal blood.

14. The use according to claim 12, wherein said target cells are tumor cells.

## Patentansprüche

1. Verfahren zur Isolierung künstlicher Chromosomen aus einer Bibliothek künstlicher Chromosomen, die frei von Wirts-DNA sind, wobei das Verfahren die folgenden Schritte umfasst:
(a) Herstellen einer Bibliothek künstlicher Chromosomen mit verdauter chromosomaler DNA durch die Verwendung eines Vektors, der eine Erkennungsstelle für ein Restriktionsenzym (x) enthält, die nicht innerhalb des Genoms des Wirts vorhanden ist, der für die Vermehrung der künstlichen Chromosomen verwendet wird;
(b) Verdauen der aus der Bibliothek künstlicher Chromosomen nach Schritt (a) hergestellten DNAs mit dem Restriktionsenzym (x) nach Vermehrung der Bibliothek; und
(c) Abtrennen der verdauten linearen künstlichen Chromosomen der Bibliothek von der unverdauten zirkulären Wirts-DNA durch die Verwendung eines Trennungsverfahrens, das es erlaubt, zwischen linearer und zirkulärer DNA zu unterscheiden.

2. Verfahren nach Anspruch 1, wobei die Bibliothek künstlicher Chromosomen eine Bibliothek von BAC-Klonen ist.

3. Verfahren nach Anspruch 1 oder 2, wobei der Vektor eine einzige Erkennungsstelle für das Restriktionsenzym (x) enthält.

4. Verfahren nach einem der Ansprüche 1 bis 3, wobei das Restriktionsenzym (x) PI-Scel ist.

5. Verfahren nach einem der Ansprüche 1 bis 4, wobei der Wirt E.coli ist.

6. Verfahren nach Anspruch 5, wobei die chromosomale DNA von Zellen einer Gewebeprobe erhalten wird.

7. Verfahren nach einem der Ansprüche 1 bis 6, wobei die chromosomale DNA aus menschlichen Zellen erhalten wird.

8. Verfahren nach einem der Ansprüche 1 bis 7, wobei das Verfahren zur Abtrennung der verdauten linearen künstlichen Chromosomen von der unverdauten zirkulären Wirts-DNA Pulsfeldgelelektrophroese (PFGE) ist.

9. Verfahren nach einem der der Ansprüche 1 bis 8, wobei in Schritt (a) die chromosomale DNA von Zellen isoliert wird, die keine bekannten numerischen Veränderungen oder Ungleichgewichte in ihrer DNA haben.

10. Verwendung künstlicher Chromosomen, die gemäß einem Verfahren nach einem der Ansprüche 1 bis 9 erhältlich sind, in einem Verfahren zur DNA-Analyse einer Probe, wobei das Verfahren für die DNA-Analyse die folgenden Schritte umfasst:
(a) Herstellen eines Microarrays mit den künstlichen Chromosomen;
(b) Hybridisieren von DNA die von (einer) Zielzelle(n) erhalten wird, mit den künstlichen Chromosomen des Microarrays; und
(c) Evaluieren der Hybridisierungsergebnisse,
wobei vor Schritt (b) die Ziel-DNA amplifiziert worden ist, wobei die DNA von einer kleinen Zellpopulation oder einer einzelnen Zelle erhalten worden ist, und wobei Schritte (b) und (c) als "vergleichende genomische Hybridisierung" ("Comparative Genomic Hybridization", CGH) durchgeführt werden.

11. Verwendung gemäß Anspruch 10, wobei die Ziel-DNA durch Adapter-Linker-PCR amplifiziert worden ist.

12. Verwendung gemäß Anspruch 10 oder 11, wobei das Verfahren für die DNA-Analyse ein Verfahren für das Nachweisen numerischer Veränderungen und/oder Ungleichgewichte in der DNA einer Probe ist, wobei die Zielzellen Zellen sind, die im Verdacht stehen, numerische Veränderungen und/oder Ungleichgewichte in ihrer DNA zu haben.

13. Verwendung gemäß einem der Ansprüche 10 bis 12 für die Untersuchung winziger Mengen von ex vivo-DNA von premalignen Läsionen oder sehr kleinen Tumoren, von Biopsien vor und nach systemischer Therapie, archivierten Tumorproben von Patienten mit bekanntem klinischem Ergebnis, für die Analyse einzelner metastatischer Vorläuferzellen oder von fetalen Zellen, die im mütterlichen Blut zirkulieren.

14. Verwendung gemäß Anspruch 12, wobei die Zielzellen Tumorzellen sind.

## Revendications

1. Procédé d'isolement de chromosomes artificiels à partir d'une banque de chromosomes artificiels qui sont dépourvus d'ADN de l'hôte, ledit procédé comprenant les étapes suivantes :
(a) la préparation d'une banque de chromosomes artificiels avec de l'ADN chromosomique digéré par utilisation d'un vecteur contenant un site de reconnaissance pour une enzyme de restriction (x) qui n'est pas présent au sein du génome de l'hôte utilisé pour la propagation des chromosomes artificiels ;
(b) la digestion des ADNs préparés à partir de la banque de chromosomes artificiels de l'étape (a) avec l'enzyme de restriction (x) après la propagation de la banque ; et
(c) la séparation des chromosomes artificiels linéaires digérés de la banque de l'ADN circulaire non digéré de l'hôte par utilisation d'un procédé de séparation permettant de faire la distinction entre de l'ADN linéaire et circulaire.

2. Procédé selon la revendication 1, dans lequel ladite banque de chromosomes artificiels est une banque de clones BAC.

3. Procédé selon la revendication 1 ou 2, dans lequel ledit vecteur contient un seul site de reconnaissance pour l'enzyme de restriction (x).

4. Procédé selon l'une quelconque des revendications 1 à 3, dans lequel ladite enzyme de restriction (x) est PI-SceI.

5. Procédé selon l'une quelconque des revendications 1 à 4, dans lequel ledit hôte est *E. coli.*

6. Procédé selon la revendication 5, dans lequel ledit ADN chromosomique est obtenu à partir de cellules d'un échantillon tissulaire.

7. Procédé selon l'une quelconque des revendications 1 à 6, dans lequel ledit ADN chromosomique est obtenu à partir de cellules humaines.

8. Procédé selon l'une quelconque des revendications 1 à 7, dans lequel ledit procédé de séparation des chromosomes artificiels linéaires digérés de l'ADN circulaire non digéré de l'hôte est une électrophorèse sur gel en champ pulsé (PFGE).

9. Procédé selon l'une quelconque des revendications 1 à 8, dans lequel dans l'étape (a) l'ADN chromosomique est isolé de cellules qui ne présentent aucun changement numérique ou déséquilibre connu dans leur ADN.

10. Utilisation de chromosomes artificiels pouvant être obtenus selon un procédé selon l'une quelconque des revendications 1 à 9 dans un procédé d'analyse de l'ADN d'un échantillon, où ledit procédé d'analyse de l'ADN comprend les étapes suivantes :
(a) la préparation d'une micropuce avec les chromosomes artificiels ;
(b) l'hybridation de l'ADN obtenu à partir d'une ou plusieurs cellule(s) cible(s) avec les chromosomes artificiels de ladite micropuce ; et
(c) l'évaluation des résultats de l'hybridation,
où avant l'étape (b), l'ADN cible a été amplifié, où ledit ADN a été obtenu à partir d'une petite population de cellules ou d'une seule cellule et où les étapes (b) et (c) sont réalisées en tant que « Hybridation Génotypique Comparative » (CGH).

11. Utilisation selon la revendication 10, où ledit ADN cible a été amplifié par PCR après ligature d'un adaptateur.

12. Utilisation selon la revendication 10 ou 11, où ledit procédé d'analyse de l'ADN est un procédé permettant de détecter des changements numériques et/ou des déséquilibres dans l'ADN d'un échantillon, où lesdites cellules cibles sont des cellules suspectées de présenter des changements numériques et/ou des déséquilibres dans leur ADN.

13. Utilisation selon l'une quelconque des revendications 10 à 12, pour l'étude de minuscules quantités d'ADN *ex vivo* provenant de lésions prémalignes ou de très petites tumeurs, de biopsies avant et après un traitement systémique, d'échantillons tumoraux archivés de patients dont l'issue clinique est connue, de l'analyse de cellules précurseurs métastasiques simples ou de cellules foetales circulantes dans le sang maternel.

14. Utilisation selon la revendication 12, où lesdites cellules cibles sont des cellules tumorales.
